# FASCICULE DE BREVET EUROPEEN

(11) **EP 1 068 855 B1**
(45) Date de publication et mention de la délivrance du brevet: **10.04.2002**
(21) Numéro de dépôt: 00401990.7
(22) Date de dépôt: 10.07.2000
(51) Int. Cl.: A61K 7/027, A61K 7/032

(54) **Composition à phase grasse liquide gélifiée par un polyamide à groupements ester terminaux**
Gelzubereitung mit einer flüssigen Fettphase enhaltend ein Polyamid mit Ester-Endgruppen
Composition with a liquid fatty phase gelified by a polyamide having ester end-groups

(30) Priorité: 15.07.1999 FR 9909176; 24.01.2000 FR 0000922
(43) Date de publication de la demande: 17.01.2001
(73) Titulaire: L'OREAL, 75008 Paris (FR)
(72) Inventeur: Ferrari, Véronique, 94700 Maisons-Alfort (FR); Simon, Pascal, 94400 Vitry-sur-Seine (FR)
(74) Mandataire: Lhoste, Catherine

(56) Documents cités:
- EP-A- 0 602 905
- WO-A-95/11000
- WO-A-97/25960
- US-A- 3 148 125
- US-A- 4 552 693
- US-A- 5 437 859
- US-A- 5 500 209
- US-A- 5 603 925
- US-A- 5 783 657

## Description

La présente invention se rapporte à une composition de soin et/ou de traitement et/ou de maquillage de la peau, y compris du cuir chevelu, et/ou des lèvres du visage des êtres humains, contenant une phase grasse liquide gélifiée par un polymère particulier et se présentant notamment sous forme d'un stick de rouge à lèvres, dont l'application conduit à un dépôt brillant et non-migrant.

Dans les produits cosmétiques ou dermatologiques, il est courant de trouver une phase grasse liquide structurée, à savoir gélifiée et/ou rigidifiée ; ceci est notamment le cas dans les compositions solides comme les déodorants, les baumes et les rouges à lèvres, les produits anti-cerne et les fonds de teint coulés. Cette structuration est obtenue à l'aide de cires ou de charges. Malheureusement, ces cires et charges ont tendance à matifier la composition, ce qui n'est pas toujours souhaitable en particulier pour un rouge à lèvres ; en effet, les femmes sont toujours à la recherche d'un rouge à lèvres sous forme de bâton déposant un film de plus en plus brillant.

Par phase grasse liquide, au sens de la demande, on entend une phase grasse liquide à température ambiante (25°C) et pression atmosphérique (760mm de Hg), composée d'un ou plusieurs corps gras liquides à température ambiante, appelés aussi huiles, compatibles entre eux.

La structuration de la phase grasse liquide permet en particulier de limiter son exsudation des compositions solides et, en plus, de limiter, après dépôt sur la peau ou les lèvres, la migration de cette phase dans les rides et ridules ce qui est particulièrement recherché pour un rouge à lèvres. En effet, une migration importante de la phase grasse liquide, chargée de matières colorantes, conduit à un effet inesthétique autour des lèvres, accentuant particulièrement les rides et les ridules. Cette migration est souvent citée par les femmes comme un défaut majeur des rouges à lèvres classiques.

La brillance est liée pour l'essentiel à la nature de la phase grasse liquide. Ainsi, il est possible de diminuer le taux de cires et de charges de la composition pour augmenter la brillance d'un rouge à lèvres mais alors, la migration de la phase grasse liquide augmente. Autrement dit, le taux de cires et de charges nécessaires à la réalisation d'un stick sont un frein à la brillance du dépôt.

Le demandeur a trouvé que la perte de brillance d'un stick en présence de cires était liée à la structure cristalline anisotrope de ces composés. Il a donc envisagé la fabrication d'un stick, sans cire.

L'invention a justement pour objet une composition de soin et/ou de maquillage et/ou de traitement de la peau et/ou des lèvres du visage permettant de remédier à ces inconvénients.

De façon surprenante, le demandeur a trouvé que l'utilisation de polymères particuliers permettait de structurer, même en l'absence de cire, les phases grasses liquides sous forme de stick dont l'application sur les lèvres conduisait à un film brillant et non migrant.

L'invention s'applique non seulement aux produits de maquillage des lèvres comme les rouges à lèvres, les crayons à lèvres, mais aussi aux produits de soin et/ou de traitement de la peau, y compris du cuir chevelu, et des lèvres, comme les crèmes de soin journalier et de protection solaire de la peau du visage ou des lèvres, aux produits de maquillage de la peau, aussi bien du visage que du corps humain, comme les fonds de teints notamment coulés en stick ou en coupelle, les produits anti-cerne et les produits de tatouage éphémère, aux produits d'hygiène corporelle comme les déodorants notamment en stick, et aux produits de maquillage des yeux comme les eye-liners en particulier sous forme de crayon et les mascaras notamment sous forme de pain.

De façon plus précise, l'invention a pour objet une composition structurée contenant au moins une phase grasse liquide structurée par au moins un polymère à squelette polyamide comportant au moins un groupement terminal à chaîne alkyle ou alcényle ayant au moins 4 atomes de carbone, lié au squelette par un groupe ester, ce polymère étant associé à au moins un composé amphiphile liquide à température ambiante, de valeur de HLB inférieure à 8.

HLB représente la balance hydrophile/lipophile. Selon l'invention, on peut utiliser un ou plusieurs composé amphiphiles liquides à température ambiante (25°C) et pression atmosphérique. De préférence, ce composé amphiphile ou ce mélange de composés amphiphiles présente une valeur de HLB allant de 1 à 7 et mieux allant de 1 à 5 et mieux de 3 à 5. Ce ou ces composés amphiphiles ont pour but de renforcer les propriétés structurantes du polymère à hétéroatome, de faciliter la mise en oeuvre et d'améliorer la capacité à déposer du stick.

Par "au moins un groupement terminal", on entend un ou plusieurs (deux) groupements terminaux. Par "polyamide", on entend au moins 2 motifs de répétition amide.

Les polymères de la composition de l'invention présentent du fait de leur chaîne alkyle ou alcényle en bout de squelette polyamide, une bonne solubilité dans les huiles (à savoir les composés liquides, non miscibles dans l'eau) et conduisent donc à des compositions macroscopiquement homogènes même avec un taux élevé (au moins 25 %) de polymère, contrairement aux polymères de l'art antérieur exempts de chaîne alkyle ou alcényle en bout de squelette polyamide. Des phases structurées par des polymères à squelette polyamide pour des applications cosmétiques, sont décrites dans US 5 500 209 et US 5 783 657.

Avantageusement, les polyamides faisant partie de l'invention sont des polymères résultant d'une polycondensation entre un diacide carboxylique à au moins 32 atomes de carbone (ayant notamment de 32 à 44 atomes de carbone) avec une diamine ayant au moins 2 atomes de carbone (ayant notamment de 2 à 36 atomes de carbone). Le diacide est de préférence un dimère d'acide gras ayant au moins 16 atomes de carbone comme l'acide oléïque, linoléïque, linolénïque. La diamine est de préférence l'éthylène diamine, l'hexylène diamine, l'hexaméthylène diamine, le phénylène diamine, l'éthylène triamine et encore mieux l'éthylène diamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple à 18 atomes de carbone.

La composition de l'invention peut se présenter sous forme de pâte, de solide, de crème. Elle peut être une émulsion huile-dans-eau ou eau-dans-huile, un gel anhydre, solide ou souple. De préférence, elle se présente sous forme de gel anhydre translucide ou transparent, et plus spécialement sous forme anhydre transparent, coulé en stick ou en coupelle.

De façon avantageuse, le polymère de la composition de l'invention comprend une masse moléculaire moyenne en poids allant de 1 000 à 10 000 et mieux de 2 000 à 8 000.

Le polymère structurant selon l'invention est un solide, non déformable à température ambiante (25°C) et pression atmosphérique (760mm de Hg). Il est capable de structurer la composition sans l'opacifier.

Les polymères structurant ont avantageusement une température de ramollissement supérieure à 70°C et pouvant aller jusqu'à 190°C. De préférence, ils présentent une température de ramollissement allant de 80 à 130°C et mieux de 80 à 105°C. Cette température de ramollissement est plus basse que celle des polymères structurants connus, ce qui facilite leur mise en oeuvre et limite la détériorisation de la phase grasse liquide.

De façon avantageuse, les groupements ester du polymère représentent de 10 à 50 % du nombre total des groupements ester et amide et mieux de 20 à 35 %.

Ces polymères sont plus spécialement ceux décrits dans le document US-A-5783657 de la société Union Camp. Chacun de ces polymères satisfait notamment à la formule (I) suivante : 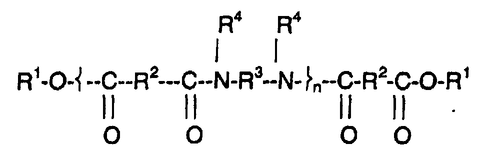 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₄₂ à condition que 50 % au moins des groupes R² représentent un groupe hydrocarboné en C₃₀ à C₄₂; R³ représente à chaque occurrence indépendamment un groupe organique pourvu d'au moins 2 atomes de carbone, d'atomes d'hydrogène et optionnellement d'un ou plusieurs atomes d'oxygène ou d'azote et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

En particulier, n représente avantageusement un nombre entier de 1 à 5. De préference R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux 75 % au moins des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou éventuellement un groupe polyoxyalkyléné et R⁴ représente un atome d'hydrogène.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Selon l'invention, la structuration de la phase grasse liquide est obtenue à l'aide d'un ou plusieurs polymères de formule (I). En général, les polymères de formule (I) se présentent sous forme de mélanges de polymères, ces mélanges pouvant en outre contenir un produit de synthèse correspondant à un composé de formule (I) avec n valant 0, c'est-à-dire un diester.

A titre d'exemple de polymère structurant utilisable dans la composition selon l'invention, on peut citer les produits commerciaux vendus par la société Bush Boake Allen sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne d'environ 6000. Les groupes esters terminausx résultent de l'estérification des terminaisons d'acide restantes par l'alcool cétylique, stéarylique ou leurs mélanges (appelés aussi alcool cétylstéarylique).

Le ou les composés amphiphiles utilisables dans la composition de l'invention comprennent une partie lipophile liée à une partie polaire, la partie lipophile comportant une chaîne carbonée ayant au moins 8 atomes de carbone, notamment de 16 à 32 atomes de carbone et mieux de 18 à 28 atomes de carbone. De préférence, la partie polaire de ce ou ces composé amphiphiles est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés. En particulier, le composé amphiphile est choisi parmi les hydroxystéarates, les oléates, les isostéarates du glycérol du sorbitan ou du méthylglucose ou les alcools gras ramifiés en C₁₂ à C₂₆ comme l'octyldodécanol et leurs mélanges. Parmi les esters, on préfère les monoesters et les mélanges de mono- et de di-esters.

La structuration ou gélification des huiles (de façon générale de la phase grasse liquide), qui est modulable par la nature du polyamide et celles du composé amphiphile utilisées est telle que l'on peut obtenir une structure rigide sous forme d'un bâton ou d'un stick.

Le taux de composés amphiphiles et celui du polymère sont choisis selon la dureté de gel désirée et en fonction de l'application particulière envisagée. Les quantités respectives de polymère et de composé amphiphile peuvent être telles qu'elles permettent l'obtention d'un solide délitable, ne s'écoulant pas sous son propre poids, présentant en particulier une dureté allant de 20 à 2 000 g et mieux de 20 à 900 g, notamment de 20 à 600 g et par exemple de 150 à 450 g. Cette dureté peut être mesurée selon la méthode de pénétration d'une sonde dans ladite composition et en particulier à l'aide d'un analyseur de texture (par exempleTA-XT2 de chez Rhéo) équipé d'un cylindre en ébonite de 5 mm de haut et 8 mm de diamètre. La mesure de dureté est effectuée à 20°C au centre de 5 échantillons de la dite composition. Le cylindre est introduit dans chaque échantillon de composition à une pré-vitesse de 2mm/s puis à une vitesse de 0,5 mm/s et enfin à une post-vitesse de 2mm/s, le déplacement total étant de 1mm. La valeur relevée de la dureté est celle du pic maximum. L'erreur de mesure est de +/- 50 g.

La dureté peut aussi être mesurée par la méthode dite du fil à couper le beurre, qui consiste à couper un bâton de rouge à lèvres de 8,1 mm et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme) nécessaire pour couper un stick dans ces conditions. Selon cette méthode la dureté d'une composition en stick selon l'invention va de 30 à 150 g, de préférence de 30 à 120 et par exemple de 30 à 50 g.

Cette dureté est telle que la composition est autoportée et peut se déliter aisément pour former un dépôt satisfaisant sur la peau et les lèvres. En outre, avec cette dureté, la composition de l'invention sous forme coulée notamment en stick résiste bien aux chocs.

Selon l'invention, la composition sous forme de stick a le comportement d'un solide élastique déformable et souple, conférant à l'application une douceur élastique remarquable. Les compositions en stick de l'art antérieur n'ont pas cette propriété d'élasticité et de souplesse.

En pratique la quantité de polymère représente (en matière active) de 0,5 à 80 % du poids total de la composition et mieux de 5 à 40 %. La quantité de composé amphiphile représente en pratique de 0,1 % à 35 % et mieux de 2 % à 15 %.

Ces bâtons ou sticks, lorsqu'ils sont colorés et en particulier pigmentés permettent, après application, d'obtenir un dépôt brillant, homogène en couleur et ne migrant pas dans les rides et ridules de la peau, entourant en particulier les lèvres, mais aussi les yeux.

Avantageusement la phase grasse liquide structurée par le polyamide contient une quantité majoritaire, à savoir supérieure à 40 % et mieux plus de 50 % en poids, d'huile ou mélange d'huiles liquides apolaires en particulier hydrocarbonées, par rapport au poids total de la phase grasse liquide.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle, alcoxy ou phényle, pendant et/ou en bout de chaîne siliconée, groupements ayant chacun de 2 à 24 atomes de carbone; les silicones phénylées comme les phényl triméthicones, les phényl diméthicones, les phényl triméthylsiloxy diphénylsiloxanes, des diphényl diméthicones, les diphényl méthyldiphényl trisiloxanes, les 2-phényléthyl triméthylsiloxysilicates ; les hydrocarbures ou fluorocarbures linéaires ou ramifiés d'origine synthétique ou minérale, volatils ou non comme les huiles de paraffine volatiles (telles que les isoparaffines, l'isododécane) ou non volatiles et leurs dérivés, la vaseline, les polydécènes, le polyisobutène hydrogéné tel que le parléam, le squalane. De préférence, les huiles utilisées sont des huiles apolaires du type hydrocarboné d'origine minérale ou synthétique, choisies notamment parmi l'huile de parléam, les isoparaffines, le squalane et leurs mélanges.

Il est possible d'ajouter aux huiles apolaires des huiles polaires, les huiles apolaires servant notamment de cosolvant des huiles polaires.

En particulier, les huiles polaires de l'invention sont :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles ou esters de synthèse de formule R₅COOR₆ dans laquelle R₅ représente le reste d'un acide gras supérieur linéaire ou ramifié comportant de 1 à 40 et mieux de 7 à 19 atomes de carbone et R₆ représente une chaîne hydrocarbonée ramifiée contenant de 1 à 40 et mieux de 3 à 20 atomes de carbone, avec R₅ + R₆ ≥ 10 comme par exemple l'huile de Purcellin (octanoate de cétostéaryle), l'isononanoate d'isononyle, le benzoate d'alcool en C₁₂ à C₁₅, le myristate d'isopropyle, le palmitate d'éthyl 2-hexyle, des octanoates, décanoates ou ricinoléates d'alcools ou de polyalcools; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en C₈ à C₂₈ comme l'alcool oléique ;
- leurs mélanges.

La phase grasse représente, en pratique, de 5 à 99 % du poids total de la composition, de préférence de 20 à 75 %.

La composition de l'invention peut comprendre, en outre, tout additif usuellement utilisé dans le domaine concerné, choisi parmi l'eau éventuellement épaissie par un épaississant ou gélifiant de phase aqueuse, les matières colorantes, les antioxydants, les huiles essentielles, les conservateurs, les parfums, les charges, les corps gras pâteux ou cireux, les neutralisants, les polymères liposolubles, les actifs cosmétiques ou dermatologiques comme par exemple les émollients, les hydratants, les vitamines, les acides gras essentiels, les filtres solaires et leurs mélanges. Ces additifs peuvent être présents dans la composition à raison de 0 à 20% du poids total de la composition et mieux de 0 à 10%.

Bien entendu l'homme du métier veillera à choisir les éventuels additifs complémentaires et/ou leur quantité de telle manière que les propriétés avantageuses de la composition selon l'invention, à savoir brillance et non-migration notamment ne soient pas ou substantiellement pas, altérées par l'adjonction envisagée.

La composition selon l'invention peut se présenter sous la forme d'une composition dermatologique ou de soin de la peau et/ou des phanères ou sous forme d'une composition de protection solaire, d'hygiène corporelle, notamment sous forme de déodorant ou de démaquillage. Elle se présente alors notamment sous forme non colorée, contenant éventuellement des actifs cosmétiques ou dermatologiques. Elle peut alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré de maquillage de la peau, présentant éventuellement des propriétés de soin ou de traitement, et en particulier un fond de teint, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils sous forme de mascara, les sourcils et les cheveux. En particulier, la composition de l'invention peut être un produit cosmétique contenant des actifs cosmétiques et/ou dermatologiques.

Bien entendu la composition de l'invention doit être cosmétiquement ou dermatologiquement acceptable, à savoir contenir un milieu physiologiquement acceptable non toxique et susceptible d'être appliqué sur la peau ou les lèvres du visage d'êtres humains. Par cosmétiquement acceptable, on entend au sens de l'invention une composition d'aspect, d'odeur et de toucher agréables.

De préférence, la matière colorante contient essentiellement des pigments et/ou des nacres en vue d'obtenir un maquillage couvrant, c'est-à-dire ne laissant pas voir la peau, lèvres ou les phanères. Les pigments permettent, en outre, de réduire le toucher collant des compositions contrairement à des colorants solubles.

Par "pigment" (nacré ou non), il faut comprendre toute particule solide insoluble dans le milieu servant à donner et/ou modifier une couleur et/ou un aspect irisé.

Avantageusement, la composition contient une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40 % du poids total de la composition, de préférence de 1 à 35 % et mieux de 5 à 25 %.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0 à 20 % du poids de la composition et mieux de 0,1 à 6 % (si présents).

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0 à 40 %, préférence de 1 à 35 % et mieux de 2 à 25 % du poids total de la composition.

Les pigments nacrés (ou nacres) peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le ou les composé amphiphiles, les matières colorantes et les additifs puis à mélanger le tout jusqu'à l'obtention d'une solution claire, transparente. Le mélange homogène obtenu peut alors être coulé dans un moule approprié comme un moule de rouge à lèvres ou directement dans les articles de conditionnement (boîtier ou coupelle notamment).

L'invention a encore pour objet un procédé cosmétique de soin, de maquillage ou de traitement des matières kératiniques des êtres humains et notamment de la peau, des lèvres du visage et des phanères des êtres humains, comprenant l'application sur les matières kératiniques de la composition notamment cosmétique telle que définie ci-dessus.

L'invention a encore pour objet l'utilisation d'une quantité suffisante d'au moins un polymère à squelette polyamide comportant au moins un groupement terminal à chaîne alkyle ou alcényle ayant au moins 4 atomes de carbone, lié au squelette par un groupe ester et d'au moins un composé amphiphile liquide à température ambiante, de valeur de HLB inférieure à 8 pour structurer une phase grasse liquide sous forme d'un solide autoporté et par exemple de dureté allant de 20 à 2 000 g et notamment de 20 à 900 g et mieux de 20 à 600 g. Cette phase grasse est notamment celle d'une d'une composition cosmétique.

L'invention a encore pour objet l'utilisation d'une quantité suffisante d'au moins un polymère à squelette polyamide comportant au moins un groupement terminal à chaîne alkyle ou alcényle ayant au moins 4 atomes de carbone, lié au squelette par un groupe ester et d'au moins un composé amphiphile liquide à température ambiante, de valeur de HLB inférieure à 8 pour structurer une phase grasse liquide sous forme d'un solide brillant et/ou non migrant.

L'invention a encore pour objet l'utilisation d'une phase grasse liquide, structurée par un polymère à squelette polyamide comportant au moins un groupement terminal à chaîne alkyle ou alcényle ayant de 4 à 22 atomes de carbone, lié par un groupe ester, et par un composé amphiphile de valeur de HLB inférieure à 8, dans une composition cosmétique ou pour la fabrication d'une composition physiologiquement acceptable brillante et/ou non migrante.

L'invention est illustrée plus en détail dans les exemples suivants. Les pourcentages sont donnés en poids.

### Exemple 1 : Formule de rouge à lèvres

- Uniclear 80 25,0 %
- Huile de parléam 56,0 %
- Polyglyceryl-2 polyhydroxystéarate 10,0%
- Pigments (oxyde de fer brun + oxyde de titane) 9,0 %

*Préparation* : On solubilise (ou dissous) l'Uniclear 80 grâce au polyglycéryl-2 polyhydroxystéarate dans l'huile de parléam, à 100 °C, puis on ajout les pigments. L'ensemble est mélangé à l'aide d'une turbine défloculeuse (Raynerie) puis coulé dans des moules de rouge à lèvres.

On obtient un stick de rouge à lèvres homogène ayant une dureté de 425 g mesurée à l'aide de l'analyseur de texture TA-XT2 à 20 °C. Le rouge à lèvres obtenu est brillant et non migrant. Ceci a été confirmé par un test sur un panel d'experts en comparaison avec un produit brillant de l'art antérieur Rouge Absolu de Lancôme. Le rouge à lèvres de l'invention a été jugé plus brillant à l'application que celui de l'art antérieur pour l'ensemble des testeurs et moins migrant au temps 0 et au bout de 2 heures de port.

### Exemple 2 : Fard à paupières anhydre

- Uniclear 80 25,0 %
- Huile de parléam 35,1 %
- Oléate de glycéryle 31.25 %
- Pigments qsp 100 %

Ce fard à paupières sous forme de stick a été réalisé comme dans l'exemple 1. Il est brillant et non migrant.

### Contre exemple

On a reproduit l'exemple 1 de rouges à lèvres en remplaçant le polyamide Uniclear 80 par le polyamide Versamid® 930 vendu par la société Henkel, puis par le polyamide Macromeit® 6212 vendu aussi par la société Henkel, ces deux polyamides étant exempts de groupement terminal à chaîne alkyle ou alcényle à au moins 4 atomes de carbone, lié au squelette polyamide par un groupe ester.

Les produits obtenus sont totalement hétérogènes et sous forme bi-phasée. Ils n'ont nullement l'aspect et la dureté d'un stick.

## Revendications

1. Composition structurée contenant au moins une phase grasse liquide structurée par au moins un polymère à squelette polyamide comportant au moins un groupement terminal à chaîne alkyle ou alcényle ayant au moins 4 atomes de carbone, lié au squelette par un groupe ester, ce polymère étant associé à au moins un composé amphiphile liquide à température ambiante, de valeur de HLB inférieure à 8.

2. Composition selon la revendication 1, **caractérisée en ce que** les groupes ester représentent de 10 à 50 % du nombre total des groupes ester et des groupes amides.

3. Composition selon la revendication 1 ou 2, **caractérisée** en ce le polmère a une masse moléculaire moyenne en poids allant de 1000 à 10 000 et mieux de 2000 à 8000.

4. Composition selon l'une quelconque des revendications précédentes, **caractérisée en ce que** le polymère est choisi parmi les polymères de formule (I) suivante et leurs mélanges: 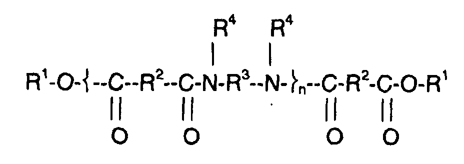 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % au moins des groupes R² représentent un groupe hydrocarboné en C₃₀ à C₄₂; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

5. Composition selon l'une quelconque des revendications précédentes, **caractérisée en ce que** les groupes ester représentent de 20 à 35 % du nombre total des groupes ester et des groupes amides.

6. Composition selon l'une des revendications 4 à 5, **caractérisée en ce que** R¹ est un groupe alkyle en C₁₂ à C₂₂ et de préférence en C₁₆ à C₂₂.

7. Composition selon l'une des revendications 4 à 6, **caractérisée en ce que** R² est un groupe hydrocarboné ayant de 10 à 42 atomes de carbone.

8. Composition selon l'une des revendications 4 à 7, **caractérisée en ce que** R³ est un groupe hydrocarboné en C₂ à C₃₆.

9. Composition selon l'une quelconque des revendications précédentes, **caractérisée en ce que** le composé amphiphile comprend une partie lipophile liée à une partie polaire, la partie lipophile comportant une chaîne carbonée ayant au moins 8 atomes de carbone de préférence de 16 à 32 atomes de carbone et mieux de 18 à 28 atomes de carbone.

10. Composition selon la revendication précédente, **caractérisée en ce que** la partie polaire est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés.

11. Composition selon l'une des revendications précédentes, **caractérisée en ce que** le composé amphiphile est choisi parmi les hydroxystéarates, les oléates, les isostéarates du glycérol, du sorbitan ou du méthylglucose et l'octyldodécanol.

12. Composition selon l'une des revendications précédentes, **caractérisée en ce que** le composé amphiphile représente de 0,1 à 35 % et mieux de 2 % à 15 % du poids total de la composition.

13. Composition selon l'une des revendications précédentes, **caractérisée en ce que** le polymère représente de 0,5 à 80 % du poids total de la composition et mieux de 5 à 40 %.

14. Composition selon l'une des revendications précédentes, **caractérisée en ce que** la phase grasse liquide contient plus de 50 % d'huile ou mélange d'huiles liquides apolaires.

15. Composition selon l'une des revendications précédentes, **caractérisée en ce que** la phase grasse contient au moins une huile hydrocarbonée d'origine minérale ou synthétique.

16. Composition selon l'une des revendications précédentes, **caractérisée en ce que** la phase grasse contient au moins une huile choisie parmi l'huile de parléam, les isoparaffines, le squalane et leurs mélanges.

17. Composition selon l'une des revendications précédentes, **caractérisée en ce que** la phase grasse liquide représente de 5 à 99 % du poids total de la composition et mieux de 20 à 75 %.

18. Composition selon l'une des revendications précédentes, **caractérisée en ce qu'**elle constitue une composition de soin et/ou de traitement et/ou de maquillage des matières kératiniques.

19. Composition selon l'une des revendications précédentes, **caractérisée en ce qu'**elle contient, en outre, au moins une matière colorante.

20. Composition selon la revendication précédente, **caractérisée en ce que** la matière colorante est choisie parmi les colorants lipophiles, les colorants hydrophiles, les pigments, les nacres et leurs mélanges.

21. Composition selon la revendication 19 ou 20, **caractérisée en ce que** la matière colorante est présente à raison de 0,01 à 40 % du poids total de la composition, de préférence de 5 à 25 %.

22. Composition selon l'une quelconque des revendications précédentes, **caractérisée en ce qu'**elle contient au moins un additif choisi parmi l'eau, les antioxydants, les huiles essentielles, les conservateurs, les neutralisants, les polymères liposolubles, les actifs cosmétiques ou dermatologiques, les charges, les parfums, les cires et leurs mélanges.

23. Composition structurée selon l'une quelconque des revendications précédentes, **caractérisée en ce qu'**elle contient un milieu cosmétiquement acceptable.

24. Composition selon l'une quelconque des revendications précédentes, **caractérisée en ce qu'**elle se présente sous forme coulée.

25. Composition selon l'une quelconque des revendications précédentes, **caractérisée en ce qu'**elle se présente sous forme de mascara, d'eye liner, de fond de teint, de rouge à lèvres, de déodorant, de produit de maquillage du corps, de produit démaquillant, de fard à paupières ou à joues, de produit anti-cerne, de shampooing ou après-shampooing traitant, de produit de protection solaire, de produit de soin du visage ou du corps.

26. Composition selon l'une quelconque des revendications précédentes, **caractérisée en ce qu'**elle contient des pigments.

27. Procédé cosmétique de soin, de maquillage ou de traitement des matières kératiniques des êtres humains, comprenant l'application sur les matières kératiniques de la composition cosmétique conforme à l'une des revendications précédentes.

28. Utilisation d'une quantité suffisante d'au moins un polymère à squelette polyamide comportant au moins un groupement terminal à chaîne alkyle ou alcényle ayant au moins 4 atomes de carbone, lié au squelette par un groupe ester et d'au moins un composé amphiphile liquide à température ambiante, de valeur de HLB inférieure à 8 pour structurer une phase grasse liquide sous forme d'un solide autoporté.

29. Utilisation selon la renvendication 28, **caractérisée en ce que** le solide a une dureté allant de 20 à 2 000 g, de préférence de 20 à 900 g.

30. Utilisation d'une phase grasse liquide, structurée par un polymère à squelette polyamide comportant au moins un groupement terminal à chaîne alkyle ou alcényle ayant de 4 à 22 atomes de carbone, lié par un groupe ester, et par un composé amphiphile de valeur de HLB inférieure à 8, pour la fabrication d'une composition physiologiquement acceptable brillante et/ou non migrante.

31. Utilisation selon la revendication 30, dans laquelle la composition est sous forme solide.

## Claims

1. Structured composition containing at least one liquid fatty phase structured with at least one polymer having a polyamide skeleton comprising at least one end group having an alkyl or alkenyl chain containing at least 4 carbon atoms, linked to the skeleton via an ester group, this polymer being combined with at least one amphiphilic compound that is liquid at room temperature, with an HLB value of less than 8.

2. Composition according to Claim 1, **characterized in that** the ester groups represent from 10% to 50% of the total number of ester groups and amide groups.

3. Composition according to Claim 1 or 2, **characterized in that** the polymer has a weight-average molecular mass ranging from 1000 to 10 000 and better still from 2000 to 8000.

4. Composition according to any one of the preceding claims, **characterized in that** the polymer is chosen from the polymers of formula (I) below, and mixtures thereof: 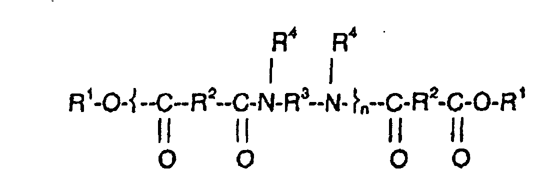 in which n denotes a number of amide units such that the number of ester groups represents
from 10% to 50% of the total number of ester and amide groups; R¹ is, independently in each case, an alkyl or alkenyl group containing at least 4 carbon atoms; R² represents, independently in each case, a C₄ to C₄₂ hydrocarbon-based group, on condition that at least 50% of the groups R² represent a C₃₀ to C₄₂ hydrocarbon-based group; R³ represents, independently in each case, an organic group containing at least 2 carbon atoms, hydrogen atoms and optionally one or more oxygen or nitrogen atoms; and R⁴ represents, independently in each case, a hydrogen atom, a C₁ to C₁₀ alkyl group or a direct bond to R³ or to another R⁴, such that the nitrogen atom to which R³ and R⁴ are both attached forms part of a heterocyclic structure defined by R⁴-N-R³, with at least 50% of the groups R⁴ representing a hydrogen atom.

5. Composition according to any one of the preceding claims, **characterized in that** the ester groups represent from 20% to 35% of the total number of ester groups and amide groups.

6. Composition according to either of Claims 4 and 5, **characterized in that** R¹ is a C₁₂ to C₂₂ and preferably C₁₆ to C₂₂ alkyl group.

7. Composition according to one of Claims 4 to 6, **characterized in that** R² is a hydrocarbon-based group containing from 10 to 42 carbon atoms.

8. Composition according to one of Claims 4 to 7, **characterized in that** R³ is a C₂ to C₃₆ hydrocarbon-based group.

9. Composition according to any one of the preceding claims, **characterized in that** the amphiphilic compound comprises a lipophilic portion linked to a polar portion, the lipophilic portion comprising a carbon-based chain containing at least 8 carbon atoms, preferably from 16 to 32 carbon atoms and better still from 18 to 28 carbon atoms.

10. Composition according to the preceding claim, **characterized in that** the polar portion is the residue of a compound chosen from alcohols and polyols containing from 1 to 12 hydroxyl groups, polyoxyalkylenes comprising at least 2 oxyalkylenated units and containing from 0 to 20 oxypropylenated units and/or from 0 to 20 oxyethylenated units.

11. Composition according to one of the preceding claims, **characterized in that** the amphiphilic compound is chosen from the hydroxystearates, oleates and isostearates of glycerol, of sorbitan or of methylglucose and octyldodecanol.

12. Composition according to one of the preceding claims, **characterized in that** the amphiphilic compound represents from 0.1% to 35% and better still from 2% to 15% of the total weight of the composition.

13. Composition according to one of the preceding claims, **characterized in that** the polymer represents from 0.5% to 80% of the total weight of the composition and better still from 5% to 40%.

14. Composition according to one of the preceding claims, **characterized in that** the liquid fatty phase contains more than 50% of apolar liquid oil or mixture of oils.

15. Composition according to one of the preceding claims, **characterized in that** the fatty phase contains at least one hydrocarbon-based oil of mineral or synthetic origin.

16. Composition according to one of the preceding claims, **characterized in that** the fatty phase contains at least one oil chosen from parleam oil, isoparaffins and squalane, and mixtures thereof.

17. Composition according to one of the preceding claims, **characterized in that** the liquid fatty phase represents from 5% to 99% of the total weight of the composition and better still from 20% to 75%.

18. Composition according to one of the preceding claims, **characterized in that** it constitutes a care composition and/or treatment composition and/or make-up composition for keratin materials.

19. Composition according to one of the preceding claims, **characterized in that** it also contains at least one dyestuff.

20. Composition according to the preceding claim, **characterized in that** the dyestuff is chosen from lipophilic dyes, hydrophilic dyes, pigments and nacres, and mixtures thereof.

21. Composition according to Claim 19 or 20, **characterized in that** the dyestuff is present in a proportion of from 0.01% to 40% of the total weight of the composition and preferably from 5% to 25%.

22. Composition according to any one of the preceding claims, **characterized in that** it contains at least one additive chosen from water, antioxidants, essential oils, preserving agents, neutralizers, liposoluble polymers, cosmetic or dermatological active agents, fillers, fragrances and waxes, and mixtures thereof.

23. Structured composition according to any one of the preceding claims, **characterized in that** it contains a cosmetically acceptable medium.

24. Composition according to any one of the preceding claims, **characterized in that** it is in cast form.

25. Composition according to any one of the preceding claims, **characterized in that** it is in the form of a mascara, an eye liner, a foundation, a lipstick, a deodorant, a body make-up product, a make-up-removing product, an eye shadow, a face powder, a concealer product, a medicated shampoo or medicated conditioner, an antisun product, or a care product for the face or the body.

26. Composition according to any one of the preceding claims, **characterized in that** it contains pigments.

27. Cosmetic care, make-up or treatment process for human keratin materials, comprising the application to the keratin materials of the cosmetic composition in accordance with one of the preceding claims.

28. Use of a sufficient amount of at least one polymer having a polyamide skeleton comprising at least one end group having an alkyl or alkenyl chain containing at least 4 carbon atoms, linked to the skeleton via an ester group, and of at least one amphiphilic compound that is liquid at room temperature, with an HLB value of less than 8, to structure a liquid fatty phase in the form of a self-supporting solid.

29. Use according to Claim 28, **characterized in that** the solid has a hardness ranging from 20 to 2000 g and preferably from 20 to 900 g.

30. Use of a liquid fatty phase, structured with a polymer having a polyamide skeleton comprising at least one end group having an alkyl or alkenyl chain containing from 4 to 22 carbon atoms, linked via an ester group, and with an amphiphilic compound with an HLB value of less than 8, for the manufacture of a physiologically acceptable glossy and/or migration-resistant composition.

31. Use according to Claim 30, in which the composition is in solid form.

## Patentansprüche

1. Strukturierte Zusammensetzung, die mindestens eine flüssige Fettphase enthält, die mit mindestens einem Polymer mit Polyamidgrundgerüst strukturiert ist, das mindestens eine endständige Gruppe mit Alkyl- oder Alkenylkette mit mindestens 4 Kohlenstoffatomen aufweist, die über eine Estergruppe an das Grundgerüst gebunden ist, wobei das Polymer mit mindestens einer bei Raumtemperatur flüssigen, amphiphilen Verbindung mit einem HLB-Wert unter 8 kombiniert ist.

2. Zusammensetzung nach Anspruch 1, **dadurch gekennzeichnet, daß** die Estergruppen 10 bis 50 % der Gesamtzahl der Esterund Amidgruppen ausmachen.

3. Zusammensetzung nach Anspruch 1 oder 2, **dadurch gekennzeichnet, daß** das Polymer ein Gewichtsmittel des Molekulargewichts im Bereich von 1000 bis 10 000 und besser 2000 bis 8000 aufweist.

4. Zusammensetzung nach einem der vorhergehenden Ansprüche, **dadurch gekennzeichnet, daß** das Polymer unter den Polymeren der folgenden Formel (I) und deren Gemischen ausgewählt ist: 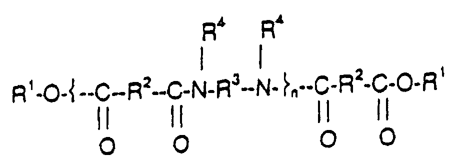 wobei n die Anzahl der Amideinheiten bezeichnet und so ausgewählt ist, daß die Anzahl der Estergruppen 10 bis 50 % der Gesamtzahl der Ester- und Amidgruppen ausmacht; die Gruppen R¹ jeweils unabhängig voneinander Alkyl- oder Alkenylgruppen mit mindestens 4 Kohlenstoffatomen bedeuten; die Gruppen R² jeweils unabhängig voneinander C₄₋₄₂-Kohlenwasserstoffgruppen bedeuten, mit der Maßgabe, daß mindestens 50 % der Gruppen R² C₃₀₋₄₂-Kohlenwasserstoffgruppen sind; die Gruppen R³ jeweils unabhängig voneinander organische Gruppen bedeuten, die mindestens zwei Kohlenstoffatome, Wasserstoffatome und gewünschtenfalls ein oder mehrere Sauerstoffatome oder Stickstoffatome enthalten; und die Gruppen R⁴ jeweils unabhängig voneinander Wasserstoff, C₁₋₁₀-Alkyl oder eine direkte Bindung zu R³ oder einer weiteren Gruppe R⁴ bedeuten, die so gebildet wird, daß das Stickstoffatom, an das die Gruppen R³ und R⁴ gleichzeitig gebunden sind, Teil einer heterocyclischen Struktur ist, die durch R⁴-N-R³ definiert ist, wobei mindestens 50 % der Gruppen R⁴ Wasserstoff bedeuten.

5. Zusammensetzung nach einem der vorhergehenden Ansprüche, **dadurch gekennzeichnet, daß** die Estergruppen 20 bis 35 % der Gesamtzahl der Ester- und der Amidgruppen ausmachen.

6. Zusammensetzung nach einem der Ansprüche 4 bis 5, **dadurch gekennzeichnet, daß** R¹ eine C₁₂₋₂₂-Alkylgruppe und vorzugsweise eine C₁₆₋₂₂-Alkylgruppe ist.

7. Zusammensetzung nach einem der Ansprüche 4 bis 6, **dadurch gekennzeichnet, daß** R² eine Kohlenwasserstoffgruppe mit 10 bis 42 Kohlenstoffatomen ist.

8. Zusammensetzung nach einem der Ansprüche 4 bis 7, **dadurch gekennzeichnet, daß** R³ eine C₂₋₃₆-Kohlenwasserstoffgruppe ist.

9. Zusammensetzung nach einem der vorhergehenden Ansprüche, **dadurch gekennzeichnet, daß** die amphiphile Verbindung einen lipophilen Bereich aufweist, der an einen polaren Bereich gebunden ist, wobei der lipophile Bereich eine kohlenstoffhaltige Kette mit mindestens 8 Kohlenstoffatomen und vorzugsweise 16 bis 32 Kohlenstoffatomen und besser 18 bis 28 Kohlenstoffatomen enthält.

10. Zusammensetzung nach dem vorhergehenden Anspruch, **dadurch gekennzeichnet, daß** der polare Bereich der Rest einer Verbindung ist, die unter den Alkoholen und Polyolen mit 1 bis 12 Hydroxygruppen und den Polyoxyalkylenen ausgewählt ist, die mindestens 2 Alkylenoxideinheiten aufweisen und 0 bis 20 Propylenoxideinheiten und/oder 0 bis 20 Ethylenoxideinheiten tragen.

11. Zusammensetzung nach einem der vorhergehenden Ansprüche, **dadurch gekennzeichnet, daß** die amphiphile Verbindung unter den Hydroxystearaten, Oleaten, Isostearaten von Glycerin, Sorbitan oder Methylglucose und Octyldodecanol ausgewählt ist.

12. Zusammensetzung nach einem der vorhergehenden Ansprüche, **dadurch gekennzeichnet, daß** die amphiphile Verbindung 0,1 bis 35 % und besser 2 bis 15 % des Gesamtgewichts der Zusammensetzung ausmacht.

13. Zusammensetzung nach einem der vorhergehenden Ansprüche, **dadurch gekennzeichnet, daß** das Polymer 0,5 bis 80 % des Gesamtgewichts der Zusammensetzung und besser 5 bis 40 % ausmacht.

14. Zusammensetzung nach einem der vorhergehenden Ansprüche, **dadurch gekennzeichnet, daß** die flüssige Fettphase mehr als 50 % eines apolaren flüssigen Öls oder Ölgemisches enthält.

15. Zusammensetzung nach einem der vorhergehenden Ansprüche, **dadurch gekennzeichnet, daß** die Fettphase mindestens ein Kohlenwasserstofföl mineralischer oder synthetischer Herkunft enthält.

16. Zusammensetzung nach einem der vorhergehenden Ansprüche, **dadurch gekennzeichnet, daß** die Fettphase mindestens ein Öl enthält, das unter Parleam, Isoparaffinen, Squalan und ihren Gemischen ausgewählt ist.

17. Zusammensetzung nach einem der vorhergehenden Ansprüche, **dadurch gekennzeichnet, daß** die flüssige Fettphase 5 bis 99 % des Gesamtgewichts der Zusammensetzung und besser 20 bis 75 % ausmacht.

18. Zusammensetzung nach einem der vorhergehenden Ansprüche, **dadurch gekennzeichnet, daß** sie eine Zusammensetzung zur Pflege und/oder zur Behandlung und/oder zum Schminken von Keratinsubstanzen ist.

19. Zusammensetzung nach einem der vorhergehenden Ansprüche, **dadurch gekennzeichnet, daß** sie ferner mindestens ein Farbmittel enthält.

20. Zusammensetzung nach dem vorhergehenden Anspruch, **dadurch gekennzeichnet, daß** das Farbmittel unter den lipophilen Farbmitteln, hydrophilen Farbmitteln, Pigmenten, Perlglanzpigmenten und deren Gemischen ausgewählt ist.

21. Zusammensetzung nach Anspruch 19 oder 20, **dadurch gekennzeichnet, daß** das Farbmittel in Mengenanteilen von 0,01 bis 40 % des Gesamtgewichts der Zusammensetzung und vorzugsweise 5 bis 25 % vorliegt.

22. Zusammensetzung nach einem der vorhergehenden Ansprüche, **dadurch gekennzeichnet, daß** sie mindestens einen Zusatzstoff enthält, der unter Wasser, Antioxidantien, etherischen Ölen, Konservierungsmitteln, Neutralisationsmitteln, fettlöslichen Polymeren, kosmetischen oder dermatologischen Wirkstoffen, Füllstoffen, Parfums, Wachsen und deren Gemischen ausgewählt ist.

23. Strukturierte Zusammensetzung nach einem der vorhergehenden Ansprüche, **dadurch gekennzeichnet, daß** sie ein kosmetisch akzeptables Medium enthält.

24. Zusammensetzung nach einem der vorhergehenden Ansprüche, **dadurch gekennzeichnet, daß** sie in gegossener Form vorliegt.

25. Zusammensetzung nach einem der vorhergehenden Ansprüche, **dadurch gekennzeichnet, daß** sie als Mascara, Eyeliner, Makeup, Lippenstift, Deodorant, Produkt zum Schminken des Körpers, Produkt zum Abschminken, Lidschatten, Wangenrouge, Produkt gegen Augenringe, Haarwaschmittel, Haarpflegespülung, Produkt zum Sonnenschutz und Produkt zur Pflege des Gesichts oder des Körpers vorliegt.

26. Zusammensetzung nach einem der vorhergehenden Ansprüche, **dadurch gekennzeichnet, daß** sie Pigmente enthält.

27. Kosmetisches Verfahren zur Pflege, zum Schminken oder zur Behandlung von menschlichen Keratinsubstanzen, das umfaßt, auf die Keratinsubstanzen eine kosmetische Zusammensetzung nach einem der vorhergehenden Ansprüche aufzutragen.

28. Verwendung einer ausreichenden Menge mindestens eines Polymers mit Polyamidgrundgerüst, das mindestens eine endständige Gruppe mit Alkyl- oder Alkenylkette mit mindestens 4 Kohlenstoffatomen enthält, die über eine Estergruppe an das Grundgerüst gebunden ist, und mindestens einer bei Raumtemperatur flüssigen, amphiphilen Verbindung mit einem HLB-Wert unter 8 zur Strukturierung einer flüssigen Fettphase in Form eines selbsttragenden Feststoffs.

29. Verwendung nach Anspruch 28, **dadurch gekennzeichnet, daß** der Feststoff eine Härte von 20 bis 2000 g und vorzugsweise 20 bis 900 g aufweist.

30. Verwendung einer flüssigen Fettphase, die mit einem Polymer mit Polyamidgrundgerüst, das mindestens eine endständige Gruppe mit Alkyl- oder Alkenylgruppe mit 4 bis 22 Kohlenstoffatomen aufweist, die über eine Estergruppe gebunden ist, und einer amphiphilen Verbindung mit einem HLB-Wert unter 8 strukturiert ist, zur Herstellung einer physiologisch akzeptablen glänzenden und/oder nicht migrierenden Zusammensetzung.

31. Verwendung nach Anspruch 30, wobei die Zusammensetzung als Feststoff vorliegt.
